(19) Europäisches Patentamt
European Patent Office
Office européen des brevets

(11) Veröffentlichungsnummer: **0 000 154**
**B1**

(12) # EUROPÄISCHE PATENTSCHRIFT

(45) Veröffentlichungstag der Patentschrift: **29.07.81**

(51) Int. Cl.³: **C 07 D 209/62,**
**A 61 K 31/40**

(21) Anmeldenummer: **78100169.8**

(22) Anmeldetag: **15.06.78**

(54) **Neue Benz(f)isoindoline, ihre Herstellung und Heilmittel welche diese Verbindungen enthalten.**

(30) Priorität: **28.06.77 CH 7916/77**
**28.06.77 CH 7917/77**

(43) Veröffentlichungstag der Anmeldung:
**10.01.79 Patentblatt 79/1**

(45) Bekanntmachung des Hinweises auf die Patenterteilung:
**29.07.81 Patentblatt 81/30**

(84) Benannte Vertragsstaaten:
**BE CH DE FR GB LU NL SE**

(56) Entgegenhaltungen:
**FR - A - 2 201 092**
**US - A - 3 973 030**

(73) Patentinhaber: **SANDOZ AG**
**Lichtstrasse 35**
**CH-4002 Basel (CH)**

(72) Erfinder: **Achini, Roland, Dr.**
**Teichstrasse 104**
**CH-4106 Therwil (CH)**
Erfinder: **Oppolzer, Wolfgang, Dr.**
**4B Chemin de la Cocuaz**
**CH-1253 Vandoeuvres (CH)**
Erfinder: **Pfenninger, Emil, Dr.**
**Schönenbuchstrasse 92**
**CH-4123 Allschwil (CH)**

**EP 0 000 154 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Benz[f]isoindoline, ihre Herstellung und Heilmittel, welche diese Verbindungen enthalten

Die Erfindung betrifft neue Verbindungen der Formel I

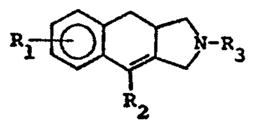

I

worin
- $R_1$ für Wasserstoff, Fluor, Chlor, Brom, Alkyl mit 1—4 C-Atomen,
- $R_2$ für Wasserstoff oder Alkyl mit 1—4 C-Atomen und
- $R_3$ für Wasserstoff oder Alkyl mit 1—2 C-Atomen

stehen, und ihre Säureadditionssalze sowie Verfahren zu ihrer Herstellung.

In der obigen Formel steht $R_2$ als Alkylgruppe besonders für Aethyl oder Methyl, ganz besonders für Methyl. Vorzugsweise bedeuten $R_2$ und $R_3$ unabhängig voneinander Wasserstoff oder Methyl.

Erfindungsgemäss gelangt man zu den Verbindungen der Formel Ia

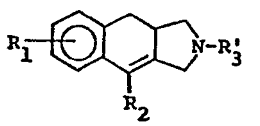

Ia

worin $R_3'$ für Alkyl mit 1—2 C-Atomen steht, und ihren Säureadditionssalzen, indem man Verbindungen der Formel II

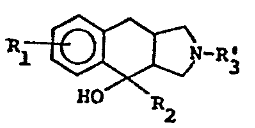

II

dehydratisiert und gewünschtenfalls die erhaltenen Verbindungen der Formel Ia in ihre Säureadditionssalze überführt. Diese Dehydratisierung kann nach an sich bekannten Methoden, z.B. in Gegenwart einer Mineralsäure wie Salzsäure oder einer starken organischen Säure wie Trifluoressigsäure oder mit Essigsäureanhydrid, Thionylchlorid oder Phosphoroxychlorid, gegebenenfalls bei erhöhter Temperatur, z.B. bei Siedetemperatur des Reaktionsgemisches, durchgeführt werden.

Erfindungsgemäss gelangt man zu den Verbindungen der Formel Ib

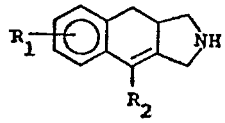

Ib

und ihren Säureadditionssalzen, indem man aus Verbindungen der Formel III

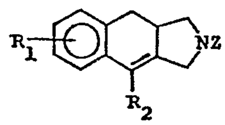

III

worin Z für eine abspaltbare Acylgruppe steht, diese Schutzgruppe entfernt und gewünschtenfalls die erhaltenen Verbindungen der Formel Ib in ihre Säureadditionssalze überführt.

Z bedeutet vorzugsweise eine $R_4SO_2$— oder $R_5CO$-Gruppe,

worin
- $R_4$ für Alkyl mit 1—4 C-Atomen, Phenyl oder p-Tolyl und
- $R_5$ für Wasserstoff, Alkyl mit 1—4 C-Atomen, $CF_3$, Phenyl, Alkoxy mit 1—4 C-Atomen, Phenoxy oder Benzyloxy

stehen.

Die erfindungsgemässe Abspaltung der Gruppe Z aus den Verbindungen der Formel III kann nach an sich bekannten Methoden durchgeführt werden.

Die Abspaltung einer $R_5CO$-Gruppe erfolgt vorzugsweise durch saure oder basische Hydrolyse.

Die basische Hydrolyse kann z.B. mit Hilfe einer 1- bis etwa 5N-Lösung eines Alkalimetallhydroxids wie Natrium-oder Kaliumhydroxid durchgeführt werden. Ein geeignetes Lösungsmittel ist z.B. ein niederes Alkanol; insbesondere Methanol und Aethanol sind geeignet. Steht $R_5CO$ für eine leicht abspaltbare Acylgruppe, z.B. die Trifluoracetylgruppe oder die Benzyloxycarbonylgruppe, so kann die Hydrolyse bei Raumtemperatur bzw. leicht erhöhter Temperatur erfolgen. Die Hydrolyse ist dann nach etwa 1/2 bis etwa 2 Stunden vollendet. Steht $R_5CO$ für eine weniger leicht abspaltbare Acylgruppe, z.B. für die Aethoxycarbonylgruppe, so arbeitet man zweckmässig unter Erwärmung, vorzugsweise unter Rückflusstemperatur des Reaktionsgemisches. Die Reaktion dauert dann etwa 10 bis 20 Stunden. Die saure Hydrolyse kann beispielsweise mit Hilfe von 2N Chlorwasserstoffsäure, zweckmässig bei erhöhter Temperatur, vorzugsweise bei Rückflusstemperatur des Reaktionsgemisches erfolgen. Steht Z für eine $R_4SO_2$-Gruppe, so kann diese Gruppe unter reduktiven Bedingungen — analog zu bekannten Methoden —, beispielsweise mit Natriumdihydro-bis-(2-methoxyäthoxy)-aluminat, oder hydrolytisch, beispielsweise mit Phenol in 40% Bromwasserstoffsäure/Essigsäure, gespalten werden.

Die erfindungsgemäss erhaltenen Verbindungen der Formel I können in Form der freien Basen oder ihrer Säureadditionssalze vorliegen. Die freien Basen können auf an sich bekannte Weise in ihre Säureadditionssalze überführt werden und umgekehrt. So können die erfindungsgemässen Verbindungen der Formel I z.B.

mit anorganischen Säuren wie Chlorwasserstoffsäure oder mit organischen Säuren wie Maleinsäure Säureadditionssalze bilden.

Die Ausgangsverbindungen der Formel II können beispielsweise wie folgt erhalten werden:

a) Alkylierung eines Amins der Formel IV

$$NC—CH_2—CH_2—NHR_3' \qquad IV$$

mit einem Cinnamylhalogenid der Formel V

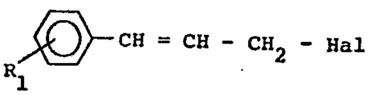

V

in Gegenwart einer Base, z.B. NaOH, oder in einem Ueberschuss des Amins der Formel IV. Die Alkylierung in Gegenwart von NaOH erfolgt vorzugsweise in einem Wasser-Methylenchlorid-Gemisch unter Zusatz eines Phasentransferkatalysators wie Benzyl-tri-(n-butyl)-ammoniumbromid.

b) Cyclisierung der erhaltenen Verbindung der Formel VI

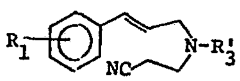

V

mit einer Base, z.B. NaH in Hexamethylphosphorsäuretriamid oder $NaOC_2H_5$ in Dimethylformamid, zu einer Verbindung der Formel VII

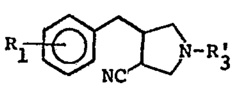

VII

c) Hydrolyse und Cyclisierung der Verbindung der Formel VII in Gegenwart einer Säure wie z.B. Polyphosphorsäure zu einer Verbindung der Formel VIII

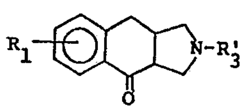

VIII

d) 1) falls $R_2$ für Wasserstoff steht: Reduktion des Ketons der Formel VIII zu dem Alkohol der Formel IXa

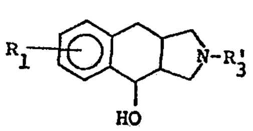

IXa

z.B. mit komplexen Metallhydriden wie $LiAlH_4$ oder $NaBH_4$ in einem geeigneten Lösungsmittel wie z.B. Aether oder Tetrahydrofuran bzw. Aethanol;

2) falls $R_2$ für Alkyl steht: Umsetzung mit einer metallorganischen Verbindung, z.B. $R_2'MgHal$ oder $R_2'Li$, worin $R_2'$ eine Alkylgruppe mit 1—4 C-Atomen bedeutet, und anschliessende Hydrolyse zu dem Alkohol der Formel IXb

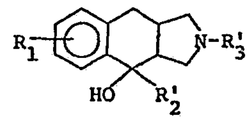

IXb

Die Verbindungen der Formeln IV und V sind bekannt oder können nach an sich bekannten Methoden hergestellt werden.

Die Ausgangsverbindungen der Formel III können z.B. durch thermische Cycloaddition einer Verbindung der Formel Xa

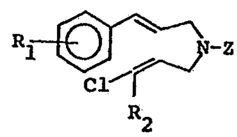

Xa

oder Xb

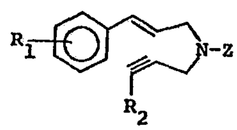

Xb

erhalten werden.

Diese thermische Cyclisierung kann in einem inerten organischen Lösungsmittel mit vorzugsweise hohem Siedepunkt, beispielsweise Dichlorbenzol, erfolgen. Man arbeitet zweckmässig unter Sauerstoffausschluss bei 160 bis 190°C.

Zu den Verbindungen der Formeln Xa und Xb gelangt man z.B., indem man eine Verbindung der Formel XI

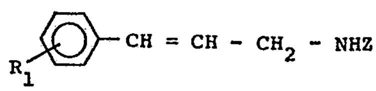

XI

mit einer Verbindung der Formel XIIa

$$R_2—C(Cl)=CH—CH_2—Hal \qquad XIIa$$

bzw. der Formel XIIb

$$R_2—C{\equiv}X—CH_2Hal \qquad XIIb$$

alkyliert, z.B. in Gegenwart von NaH in Hexamethylphosphorsäuretriamid.

Die Verbindungen der Formeln XI, XIIa und XIIb sind bekannt oder nach an sich bekannten Methoden herstellbar.

Die Verbindungen der Formel I und ihre pharmakologisch verträglichen Säureadditionssalze zeichnen sich durch interessante pharmakodynamische Eigenschaften aus und können daher als Heilmittel verwendet werden. Insbesondere zeigen die Verbindungen antiaggressive Eigenschaften. Die antiaggressiven Wirkungen zeigen sich im Tierversuch z.B. an Mäusen in einer Dämpfung des durch Isolation bedingten aggressiven Verhaltens.

Aufgrund ihrer aggressionshemmenden Eigenschaften können die Substanzen zur Behandlung von aggressiven Erregungszuständen, beispielsweise zur Dämpfung von aggressivem Verhalten von Psychopathen und Schwachsinnigen, Verwendung finden. Die zu verwendende Tagesdosis liegt bei etwa 1 bis 30 mg. Diese Dosis kann nötigenfalls in zwei bis zier Anteilen oder auch als Retardform verabreicht werden. So enthalten z.B. für orale Applikationen die Teildosen etwa 0,25 bis 15 mg der Verbindungen der Formel I neben festen oder flüssigen Trägersubstanzen.

Ausserdem besitzen die Substanzen in höheren Dosen auch zentraldämpfende Eigenschaften. Die zentraldämpfenden Wirkungen zeigen sich im Tierversuch z.B. an Mäusen bei der Messung der motorischen Aktivität im Klettertest. Aufgrund ihrer zentraldämpfenden Wirkungen können die Substanzen in der Psychiatrie zur Behandlung von Erregungszuständen Verwendung finden. Die zu verwendende Tagesdosis liegt bei etwa 10 bis 200 mg. Diese Dosis kann nötigenfalls in zwei bis vier Anteilen oder auch als Retardform verabreicht werden. So enthalten z.B. für orale Applikationen die Teildosen etwa 2,5 bis 100 mg der Verbindungen der Formel I neben festen oder flüssigen Trägersubstanzen.

Die Erfindung betrifft auch Heilmittel, die eine Verbindung der Formel I enthalten. Diese Heilmittel, beispielsweise eine Lösung ode eine Tablette, können nach bekannten Methoden, unter Verwendung der üblichen Hilfsund Trägerstoffe, hergestellt werden.

In den nachfolgenden Beispielen erfolgen alle Temperaturangaben in Celsiusgraden.

Beispiel 1

*6 - Chlor - 9,9a - dihydro - 2,4 - dimethylbenz[f]isoindolin - hydrochlorid*

Eine Lösung von 14 g 6 - Chlor - 3a,4,9,9a - tetrahydro - 2,4 - dimethylbenz[f]isoindolin - 4 - ol in 140 ml Trifluoressigsäure wird eine Stunde bei Raumtemperatur gerührt und anschliessend eingedampft. Der Rückstand wird in eiskalte wässrige Natronlauge aufgenommen, mit Methylenchlorid extrahiert, die organische Phase über Natriumsulfat getrocknet und eingedampft. Durch Kristallisation des Rückstandes aus methanolischer Salzsäure-Aether erhält man die Titelverbindung; Smp. 253—255°.

Analog erhält man unter Verwendung der entsprechenden Ausgangsverbindungen:

Beispiel 2

*9,9a - Dihydro - 2 - methylbenz[f]isoindolin - hydrochlorid*

Smp. 219—229° (Zers.)

Beispiel 3

*9,9a - Dihydro - 2,4 - dimethylbenz[f]isoindolin - hydrogenfumarat*

Smp. 205—206°

Beispiel 4

*6 - Chlor - 9,9a - dihydro - 2 - methylbenz[f]isoindolin - hydrogenfumarat*

Smp. 211—212°

Beispiel 5

*2 - Aethyl - 9,9a - dihydro - 4 - methylbenz[f]isoindolin - hydrochlorid*

Smp. 237—239° (Zers.)

Beispiel 6

*9,9a - Dihydro - 8 - methoxy - 2 - methylbenz[f]isoindolin - hydrogenfumarat*

Smp. 185—187° (Zers.)

Die im Beispiel 1 verwendete Ausgangsverbindung kann auf folgende Weise hergestellt werden:

a) Zu einem Gemisch von 42 g 3 - Methylamino - propionitril und 8,9 g Benzyl - tri - (n - butyl)ammoniumbromid in 1 Liter Methylenchlorid und 500 ml 2N Natronlauge wird unter Rühren in einer Stickstoffatmosphäre bei Raumtemperatur eine Lösung von 116 g p-Chlorcinnamylbromid in 500 ml Methylenchlorid getropft und die Emulsion 65 Stunden bei Raumtemperatur gerührt. Die organische Phase wird abgetrennt, mit Wasser gewaschen, über Natriumsulfat getrocknet und eingedampft. Man erhält 3 - [N - (p - Chlorcinnamyl) - methylamino]propionitril; Smp. des Hydrogenoxalots 143°.

b) Zu einer Suspension von 36 g Natriumhydrid (80% in Mineralöl) in 2 l Hexamethylphosphorsäuretriamid (HMPT) werden bei 0—5° unter Rühren und in einer Stickstoffatmosphäre innert $1\frac{1}{4}$ Stunden 252 g 3 - [N - (p - Chlorcinnamyl) - methylamino]propionitril in 1 Liter HMPT getropft und das Gemisch bei Raumtemperatur 16 Stunden gerührt. Dann wird das Gemisch unter Eiskühlung mit Wasser versetzt, mit Aether extrahiert, die organische Phase mit Wasser gewaschen, über Natriumsulfat getrocknet und eingedampft. Man erhält 4 - (p - Chlorbenzyl) - 1 - methylpyrrolidin - 3 - carbonitril; Smp. des Hydrogenoxalats 170—172°.

c) Zu 500 g Polyphosphorsäure werden bei 50° 25 ml Wasser, dann 50 g 4 - (p - Chlorbenzyl) - 1 - methylpyrrolidin - 3 - carbonitril getropft und das Gemisch 1 Stunde bei 125°, dann $2\frac{1}{2}$ Stunden bei 160° gerührt. Das abgekühlte Gemisch wird dann mit Eis und 1 Liter 50%-iger Natronlauge versetzt und mit Essigsäureäthylester extrahiert. Die organische Phase wird mit Wasser gewaschen, über Natriumsulfat getrocknet und eingedampft. Man erhält 6 - Chlor - 3a,4,9,9a - tetrahydro - 2 -

methylbenz[f]isoindolin - 4 - on; Smp. des Hydrogenmaleinats 148 bis 150°.

d) Zu 170 ml einer ca. 5%-igen Lösung von Methyllithium in Aether wird bei Raumtemperatur unter Rühren in einer Stickstoffatmosphäre eine Lösung von 43 g 6 - Chlor - 3a,4,9,9a - tetrahydro - 2 - methylbenz[f]-isoindolin - 4 - on in 430 ml Tetrahydrofuran getropft, das Gemisch $5\frac{1}{2}$ Stunden bei Raumtemperatur gerührt, dann mit gesättigter wässriger Ammonchloridlösung und Wasser versetzt und mit Methylenchlorid extrahiert. Die organische Phase wird über Natriumsulfat getrocknet und eingedampft. Beim Kristallisieren des Rückstandes aus Aether - Petroläther erhält man 6 - Chlor - 3a,4,9,9a - tetrahydro - 2,4 - dimethylbenz[f]isoindolin - 4 - ol; Smp. 125 bis 130°.

Die für die Verbindung des Beispiels 4 benötigte Ausgangsverbindung kann folgendermassen hergestellt werden:

e) Eine Lösung von 10 g 6 - Chlor - 3a,4,9,9a - tetrahydro - 2 - methylbenz[f]isoindolin - 4 - on in 100 ml Aethanol wird bei 0—5° mit 1,6 g Natriumborhydrid in 20 ml Aethanol versetzt, 1 Stunde bei Raumtemperatur weitergerührt und dann mit Wasser versetzt und eingedampft. Der Rückstand wird in 10%-iger Weinsäure aufgenommen, die wässrige Phase mit Aether extrahiert, mit 2N Natronlauge gestellt und mit Essigsäureäthylester extrahiert. Die Essigesterphase wird über Natriumsulfat getrocknet und eingedampft. Durch Krstallisation des Rückstandes aus Methylenchlorid - Pentan erhält man 6 - Chlor - 3a,4,9,9a - tetrahydro - 2 - methylbenz[f]isoindolin - 4 - ol; Smp. 178—182°.

Beispiel 7

*9,9a - Dihydrobenz[f]isoindolin - hydrochlorid*

Ein Gemisch von 10 g 9,9a - Dihydrobenz[f]isoindolin - 2 - trifluoracetamid und 50 ml 3N Kaliumhydroxid in Methanol wird 16 Stunden bei Raumtemperatur stehen gelassen, die Lösung eingedampft, der Rückstand in Wasser aufgenommen und mit Aether extrahiert. Die Aetherphase wird über Natriumsulfat getrocknet und eingedampft. Der Rückstand wird mit methanolischer Salzsäure eingedampft und aus Methanol-Aether kristallisiert. Man erhält die Titelverbindung vom Smp. 240—248° (Zers.).

Analog erhält man unter Verwendung der entsprechenden Ausgangsverbindungen:

Beispiel 8

*9,9a - Dihydro - 4 - methylbenz[f]isoindolin - hydrogenfumarat*

Smp. 224—226°

Beispiel 9

*9,9a - Dihydro - 6 - methylbenz[f]isoindolin*

Smp. 116—118°

Das im Beispiel 6 verwendete 9,9a - Dihydrobenz[f]isoindolin - 2 - trifluoracetamid kann auf folgende Art hergestellt werden:

a) Zu einer Suspension von 27 g Natriumhydrid in 400 ml Hexamethylphosphorsäuretriamid (HMPT) wird unter Eiskühlung und Rühren in einer Stickstoffatmosphäre eine Lösung von 250 g N-Cinnamyltrifluoracetamid in 1 Liter HMPT getropft. Nach Beendigung der Gasentwicklung wird eine Lösung von 130 g 1,3 - Dichlorpropen und 1 g Natriumjodid in 1 Liter HMPT zugetropft und das Gemisch 16 Stunden bei Raumtemperatur gerührt. Dann wird das Reaktionsgemisch auf Wasser gegossen und mit Aether extrahiert. Die über Natriumsulfat getrocknete Aetherlösung wird eingedampft und der ölige Rückstand mit Toluol an 3,5 kg Kieselgel chromatographiert. Man erhält N - (3 - Chlor - 2 - propenyl) - N - cinnamyltrifluoracetamid als Oel.

b) Eine Lösung von 267 g N - (3 - Chlor - 2 - propenyl) - N - cinnamyltrifluoracetamid in 5,5 Liter o-Dichlorbenzol wird 30 Stunden in einer Argonatmosphäre am Rückfluss zum Sieden erhitzt und anschliessend eingedampft. Der Rückstand wird mit Toluol an 6 kg Kieselgel chromatographiert. Nach Umkristallisation aus Methylenchlorid-Aether erhält man 9,9a - Dihydrobenz[f]isoindolin - 2 - trifluoracetamid vom Smp. 150—155°.

## Patentansprüche

1. Neue Verbindungen der Formel I

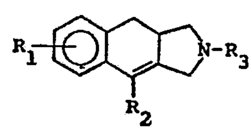

I

worin

$R_1$ für Wasserstoff, Fluor, Chlor, Brom, Alkyl mit 1—4 C-Atomen oder Alkoxy mit 1—4 C-Atomen,

$R_2$ für Wasserstoff oder Alkyl mit 1—4 C-Atomen und

$R_3$ für Wasserstoff oder Alkyl mit 1—2 C-Atomen

stehen, und ihre Säureadditionssalze.

2. Verfahren zur Herstellung der Verbindungen der Formel I und ihrer Säureadditionssalze gemäss Anspruch 1, dadurch gekennzeichnet, dass man

a) zur Herstellung der Verbindungen der Formel Ia

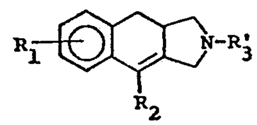

Ia

worin $R_3'$ für Alkyl mit 1—2 C-Atomen steht, Verbindungen der Formel II

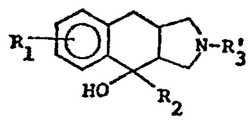

II

dehydratisiert;

b) zur Herstellung der Verbindungen der Formel Ib

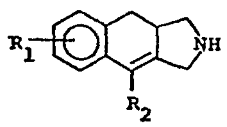

Ib

aus Verbindungen der Formel III

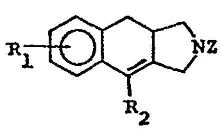

III

worin Z für eine abspaltbare Acylgruppe steht,-diese Schutzgruppe entfernt und gewünschtenfalls die erhaltenen Verbindungen der Formel Ia bzw. Ib in ihre Säureadditionssalze überführt.

3. Verbindungen der Formel Ia gemäss Anspruch 1

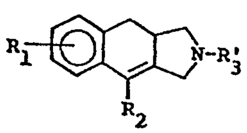

Ia

worin

$R_1$ und $R_2$ die im Anspruch 1 angegebenen Bedeutungen haben

$R_3^1$ für Alkyl mit 1—2 C-Atomen steht und ihre Säureadditionssalze.

4. Verbindungen der Formel Ib gemäss Anspruch 1

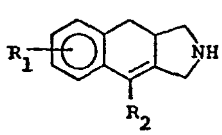

Ib

worin

$R_1$ und $R_2$ die im Anspruch 1 angegebenen Bedeutungen haben

und ihre Säureadditionssalze.

5. Die Verbindung 9,9a - Dihydro - 2,4 - dimethylbenz[f]isoindolin gemäss Anspruch 1 und ihre Säureadditionssalze.

6. Heilmittel enthaltend mindestens eine Verbindung gemäss einer der Ansprüche 1, 3, 4 und 5.

7. Eine Verbindung gemäss einer der Ansprüche 1, 3, 4 und 5 zur Verwendung als Heilmittel.

8. Eine Verbindung gemäss einer der Ansprüche 1, 3, 4 und 5 zur Verwendung bei der Behandlung von aggressiven Erregungszuständen.

9. Eine Verbindung gemäss einer der Ansprüche 1, 3, 4 und 5 zur Verwendung bei der Behandlung von Erregungszuständen.

**Claims**

1. New compounds of formula I

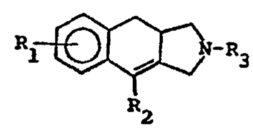

I

wherein

$R_1$ is hydrogen, fluorine, chlorine, bromine, alkyl of 1 to 4 carbon atoms or alkoxy of 1 to 4 carbon atoms.

$R_2$ is hydrogen or alkyl of 1 to 4 carbon atoms, and

$R_3$ is hydrogen or alkyl of 1 or 2 carbon atoms, and their acid addition salts.

2. Processes for the production of compounds of formula I, and their acid addition salts, as defined in claim 1, characterized in that

a) the production of compounds of formula Ia

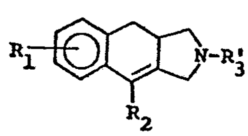

Ia

wherein $R_3'$ is alkyl of 1 or 2 carbon atoms, compounds of formula II

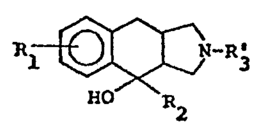

II

are dehydrated or

b) for production of compounds of formula Ib

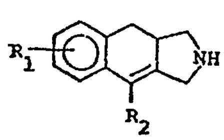

Ib

the protecting group is removed from compounds of formula III

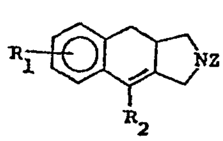

III

wherein Z is a remcvable acyl group, and if desired the resultant compounds of formula Ia or Ib are converted into acid addition salt form.

3. Compounds of formula Ia, as defined in claim 1,

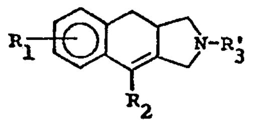

Ia

wherein
$R_1$ and $R_2$ are as defined in claim 1, and
$R_3'$ is alkyl of 1 or 2 carbon atoms,
and their acid addition salts.

4. Compounds of formula Ib as defined in claim 1

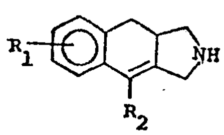

Ib

wherein
$R_1$ and $R_2$ are as defined in claim 1
and their acid addition salts.

5. The compound 9,9a - dihydro - 2,4 - dimethylbenz[f]isoindoline according to claim 1, and its acid addition salts.

6. A medicament containing a compound of any one of the claims 1, 3, 4 and 5.

7. A compound according to any one of claims 1, 3, 4 and 5 for use as a medicament.

8. A compound according to any one of claims 1, 3, 4 and 5 for use in the treatment of aggressive excitation conditions.

9. A compound according to any one of claims 1, 3, 4 and 5 for use in the treatment of excitation conditions.

## Revendications

1. Nouveaux composés de formule I

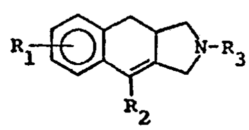

I

dans laquelle
$R_1$ signifie l'hydrogène, le fluor, le chlore, le brome, un groupe alkyle contenant de 1 à 4 atomes de carbone ou alcoxy contenant de 1 à 4 atomes de carbone.
$R_2$ signifie l'hydrogène ou un groupe alkyle contenant de 1 à 4 atomes de carbone et
$R_3$ signifie l'hydrogène ou un groupe alkyle contenant 1 ou 2 atomes de carbone,
et leurs sels d'addition d'acides.

2. Procédé de preparation des composés de formule I et de leurs sels d'addition d'acides selon la revendication 1, caractérisé en ce que

a) pour préparer les composés de formule Ia

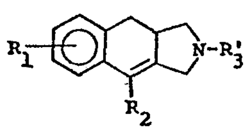

Ia

dans laquelle $R_3'$ signifie un groupe alkyle contenant 1 ou 2 atomes de carbone, on déshydrate des composés de formule II

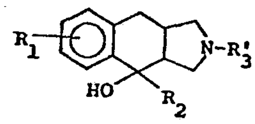

II

b) pour préparer les composés de formule Ib

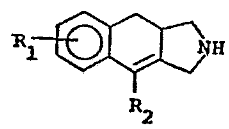

Ib

à partir de composés de formule III

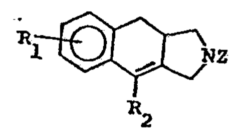

III

dans laquelle Z signifie un groupe acyle susceptible d'être séparé, on élimine ce groupe protecteur
et si on le désire, on transforme les composés de formule Ia respectivement Ib en leurs sels d'addition d'acides.

3. Composés de formule Ia selon la revendication 1

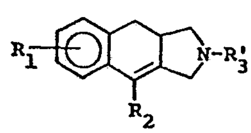

Ia

dans laquelle
$R_1$ et $R_2$ ont les significations données à la revendication 1,
$R_3'$ signifie un groupe alkyle contenant 1 ou 2 atomes de carbone
et leurs sels d'addition d'acides.

4. Composés de formule Ib selon la revendication 1

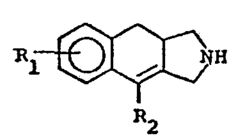

Ib

dans laquelle $R_1$ et $R_2$ ont les significations données à la revendication 1 et leurs sels d'addition d'acides.

5. Le composé 9,9a - dihydro - 2,4 - diméthylbenz[f]isoindoline selon la revendication 1 et ses sels d'addition d'acides.

6. Médicament contenant au moins un composé selon l'une des revendications 1, 3, 4 et 5.

7. Un composé selon l'une des revendications 1, 3, 4 et 5 pour l'utilisation en tant que médicament.

8. Un composé selon l'une des revendications 1, 3, 4 et 5 pour l'utilisation dans le traitement d'états d'excitation aggressifs.

9. Un composé selon l'une des revendications 1, 3, 4 et 5 pour l'utilisation dans le traitement d'états d'excitation.